# EUROPEAN PATENT APPLICATION

(11) **EP 1 715 060 A1**
(43) Date of publication of application: **25.10.2006**
(21) Application number: 05704386.1
(22) Date of filing: 28.01.2005
(51) Int. Cl.: C12Q 1/60, C12Q 1/26, C12Q 1/28, C12Q 1/32, C12Q 1/44, G01N 33/92

(54) **METHOD, REAGENT, AND KIT FOR DETERMINING CHOLESTEROL IN VERY-LOW-DENSITY LIPOPROTEIN REMNANT (VLDL REMNANT)**

(30) Priority: 29.01.2004 JP 2004021478
(71) Applicant: KYOWA MEDEX CO., LTD., Chuo-ku, Tokyo 104-6004 (JP)
(72) Inventor: MIYAUCHI, Kazuhito, c/o Kyowa Medex Co., Ltd., Tokyo 104-6004 (JP)
(74) Representative: Vossius & Partner
(86) International application number: PCT/JP2005/001626
(87) International publication number: WO 2005/073401

(57) **Abstract**

According to the present invention, esterified and free cholesterol in very low-density lipoprotein remnant (hereinafter collectively referred to as very low-density lipoprotein remnant cholesterol) in a sample is determined in an aqueous medium containing the sample in the presence of a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase or cholesterol dehydrogenase to act specifically on very low-density lipoprotein remnant cholesterol.

## Description

### Technical Field

The present invention relates to a method, a reagent and a kit for the quantitative determination of cholesterol in very low-density lipoprotein remnant (VLDL remnant) in a sample.

### Background Art

Blood contains various kinds of lipoproteins. These lipoproteins are classified into chylomicron (hereinafter abbreviated as CM), very low-density lipoprotein (hereinafter abbreviated as VLDL), low-density lipoprotein (hereinafter abbreviated as LDL) and high-density lipoprotein (hereinafter abbreviated as HDL) according to their specific gravity. Each class of lipoprotein has its specific ratio of constituents such as cholesterol, triglycerides, phospholipids and proteins and has a different activity in vivo.

In addition to these lipoproteins, blood also contains remnant lipoproteins that are formed in the process of metabolism and decomposition of lipoproteins by the action of lipoprotein lipase. Remnant lipoproteins are classified into chylomicron remnant (hereinafter abbreviated as CM remnant) and very low-density lipoprotein remnant (hereinafter abbreviated as VLDL remnant). Intermediate-density lipoprotein (hereinafter abbreviated as IDL), that is included in VLDL remnant, is lipoprotein having a specific gravity of 1.006 to 1.019 and is specifically called VLDL remnant.

Remnant lipoproteins are lipoproteins that are rich in triglycerides (TG) and relatively rich in apoE and cholesterol. CM remnant has apoB-48 and apoE as major apoproteins, and VLDL remnant has apoB-100 and apoE as major apoproteins.

In a healthy person, remnant lipoproteins are rapidly metabolized and incorporated into receptors in the liver. However, metabolic disorders and daily bad habits such as overeating and lack of exercise bring about an increase in remnant lipoproteins retained in blood, and these remnant lipoproteins retained in blood cause type III hyperlipidemia, etc., which leads to the onset of arteriosclerosis with a high probability. At the onset of arteriosclerosis, remnant lipoproteins retained in blood are considered to be easily incorporated into macrophages of arterial walls and to form initial lesions. Thus, remnant lipoproteins are considered to be one of the risk factors for arteriosclerotic diseases.

CM remnant having apoB-48 as a major apoprotein is considered to be incorporated via an LDL receptor-related protein (LRP) of the liver. VLDL remnant having apoB-100 as a major apoprotein is considered to be incorporated via an LDL receptor of the liver. Recently, the VLDL receptor pathway has been reported as one of the new pathways for foam cell formation from macrophages by remnant lipoproteins. That is, it is reported that the expression of the VLDL receptor is enhanced in the process of differentiation from monocyte to macrophage and the remnant lipoproteins cause foam cell formation from macrophages via the VLDL receptor.

The methods for the quantitative determination of remnant lipoproteins or cholesterol in remnant lipoproteins so far reported include electrophoresis methods, ultracentrifugation methods, high performance liquid chromatography (HPLC) methods and immunoadsorption methods. As the electrophoresis methods, for example, polyacrylamide gel electrophoresis (PAGE) method and agarose gel electrophoresis method have been reported. The polyacrylamide gel electrophoresis (PAGE) method employed in the qualitative analysis of remnant lipoproteins is a method for the detection of the mid-band which is an abnormal band recognized between VLDL and LDL. The agarose gel electrophoresis method also employed in the qualitative analysis of remnant lipoproteins is effectively used especially for the diagnosis of type III hyperlipidemia. In type III hyperlipidemia, β-VLDL which moves to the β-position is confirmed.

An example of ultracentrifugation method is a method which comprises separating intermediate-density lipoprotein (hereinafter abbreviated as IDL) which is lipoprotein having a specific gravity of 1.006 to 1.019 by ultracentrifugation and determining cholesterol in the obtained IDL (hereinafter abbreviated as IDL-C). However, operations of this ultracentrifugation method are very complicated.

Examples of HPLC methods include a method for the analysis of remnant-like lipoproteins (hereinafter abbreviated as RLP) in a serum sample which comprises separating CM and VLDL in serum by HPLC, and analyzing the amount of cholesterol in the separated CM and that in the separated VLDL (Japanese Published Unexamined Patent Application No. 105876/96), and a method for the analysis of RLP in a serum sample which comprises separating lipoproteins other than RLP in a human serum sample by an antibody column for separating lipoproteins filled with packings for the separation of lipoproteins prepared by immobilizing anti-human apoA-I monoclonal antibody which does not recognize apoB-48 and anti-human apoB-100 monoclonal antibody which does not recognize apoB-48 on a nonporous insoluble carrier, and then analyzing the amount of cholesterol in RLP which is lipoproteins that were not trapped (Japanese Published Unexamined Patent Application No. 105875/96). The former method is a method based on a good correlation between the total amount of CM cholesterol and VLDL cholesterol and the amount of cholesterol in RLP as measured by immunoadsorption method. In these patent publications, the term "RLP" is used to mean abnormal lipoproteins formed by decomposition of CM, VLDL, etc. with lipoprotein lipase.

Recently, a method for the determination of cholesterol in RLP by immunoadsorption method was developed. RLP as used herein refers to lipoproteins rich in triglycerides. According to this method, RLP is separated from serum by immunoaffinity chromatography using affinity gel containing anti-apoA-I monoclonal antibody and specific anti-apoB-100 monoclonal antibody which does not recognize apoB-48, and cholesterol contained in the separated RLP is determined. A reagent for the determination of cholesterol in RLP applied in this method is commercially available from JIMRO Co., Ltd. (product: RLP-cholesterol "JIMRO" II) [Clinical Chemistry, the American Association for Clinical Chemistry, Vol. 48, p. 217-219 (2002)].

With respect to the method for the determination of cholesterol in RLP, there is also a report on a method which comprises allowing cholesterol esterase and cholesterol oxidase or cholesterol dehydrogenase and an enzyme which hydrolyzes a phospholipid to act on a sample in the presence of a polyoxyalkylene derivative and a polyoxyethylene-polyoxypropylene copolymer or its derivative, and determining the formed hydrogen peroxide or reduced coenzyme (Japanese Published Unexamined Patent Application No. 231597/2001). A good correlation is recognized between the amount of cholesterol in a sample as determined using the reagent for the determination of Example 1 of this patent publication and the amount of cholesterol in RLP in a sample as determined using RLP-cholesterol "JIMRO" II which is a reagent for the determination available from JIMRO Co., Ltd. However, it has been reported that RLP-cholesterol "JIMRO" II is also reactive to cholesterol in VLDL which is not classified into remnant lipoproteins in a sample.

Recently, there has been a report on the analysis of remnant lipoproteins by a combination of ultracentrifugation and gel filtration ["Arteriosclerosis", Japan Atherosclerosis Society, Vol. 29, p. 235 (Title No. 98) (2001)].

### Disclosure of the Invention

An object of the present invention is to provide a simple and rapid method, a reagent and a kit for the quantitative determination of cholesterol in VLDL remnant (hereinafter referred to as VLDL remnant cholesterol and abbreviated as VLDL remnant-C) which is one of the risk factors for arteriosclerotic diseases.

The present invention relates to the following (1) to (23).
(1) A method for quantitatively determining esterified and free cholesterol in very low-density lipoprotein remnant (hereinafter collectively referred to as very low-density lipoprotein remnant cholesterol) in a sample, which comprises: in the presence of a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase or cholesterol dehydrogenase to act specifically on very low-density lipoprotein remnant cholesterol, allowing, in an aqueous medium containing the sample, (c) cholesterol esterase and cholesterol oxidase or (d) in the presence of oxidized coenzyme, cholesterol esterase and cholesterol dehydrogenase to act on cholesterol in very low-density lipoprotein remnant in the sample to form hydrogen peroxide or reduced coenzyme; and determining the formed hydrogen peroxide or reduce coenzyme.
(2) The method according to (1), wherein the very low-density lipoprotein remnant is intermediate-density lipoprotein.
(3) The method according to (1) or (2), wherein the enzymatic reactions are carried out in the presence of cyclodextrin or its derivative and/or albumin.
(4) The method according to (3), wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.
(5) The method according to any of (1) to (4), wherein the polyoxyethylene-polyoxyalkylene alkylaryl ether is a polyoxyethylene-polyoxypropylene alkylphenyl ether.
(6) The method according to any of (1) to (5), wherein the determination of hydrogen peroxide is carried out by subjecting the formed hydrogen peroxide to reaction with a chromogen in the presence of peroxidase to form a dye and determining the formed dye.
(7) The method according to any of (1) to (5), wherein the determination of reduced coenzyme is carried out by measuring the absorbance of the reaction solution.
(8) A reagent for the quantitative determination of very low-density lipoprotein remnant cholesterol in a sample comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase to act specifically on very low-density lipoprotein remnant cholesterol, cholesterol esterase and cholesterol oxidase.
(9) The reagent according to (8), further comprising a reagent for the determination of hydrogen peroxide.
(10) A reagent for the quantitative determination of very low-density lipoprotein remnant cholesterol comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol dehydrogenase to act specifically on very low-density lipoprotein remnant cholesterol, cholesterol esterase, cholesterol dehydrogenase and oxidized coenzyme.
(11) The reagent according to (10), further comprising a reagent for the determination of reduced coenzyme.
(12) The reagent according to any of (8) to (11), wherein the very low-density lipoprotein remnant cholesterol is intermediate-density lipoprotein.
(13) The reagent according to any of (8) to (12), further comprising cyclodextrin or its derivative and/or albumin.
(14) The reagent according to (13), wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.
(15) The reagent according to any of (8) to (14), wherein the polyoxyethylene-polyoxyalkylene alkylaryl ether is a polyoxyethylene-polyoxypropylene alkylphenyl ether.
(16) A kit for the quantitative determination of very low-density lipoprotein remnant cholesterol in a sample which comprises a first reagent comprising one kind of surfactant selected from the group consisting of a polyoxyethylene-polyoxybutylene copolymer, a polyoxyethylene styrenated-phenyl ether and a polyoxyalkylene long-chain alkyl ether which is capable of causing cholesterol esterase and cholesterol oxidase to act specifically on very low-density lipoprotein remnant cholesterol when used in combination with a polyoxyethylene-polyoxyalkylene alkylaryl ether, and a second reagent comprising cholesterol esterase and cholesterol oxidase, said kit further comprising a polyoxyethylene-polyoxyalkylene alkylaryl ether allowing cholesterol esterase and cholesterol oxidase to act specifically on very low-density lipoprotein remnant cholesterol when used in combination with said one kind of surfactant selected from the group consisting of a polyoxyethylene-polyoxybutylene copolymer, a polyoxyethylene styrenated-phenyl ether and a polyoxyalkylene long-chain alkyl ether in at least one of the first reagent and the second reagent, and further comprising a reagent for the determination of hydrogen peroxide in at least one of the first reagent and the second reagent.
(17) A kit for the quantitative determination of very low-density lipoprotein remnant cholesterol in a sample which comprises a first reagent comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase to act specifically on very low-density lipoprotein remnant cholesterol, and a second reagent comprising cholesterol esterase and cholesterol oxidase, said kit further comprising a reagent for the determination of hydrogen peroxide in at least one of the first reagent and the second reagent.
(18) A kit for the quantitative determination of very low-density lipoprotein remnant cholesterol in a sample which comprises a first reagent comprising one kind of surfactant selected from the group consisting of a polyoxyethylene-polyoxybutylene copolymer, a polyoxyethylene styrenated-phenyl ether and a polyoxyalkylene long-chain alkyl ether which is capable of causing cholesterol esterase and cholesterol dehydrogenase to act specifically on very low-density lipoprotein remnant cholesterol when used in combination with a polyoxyethylene-polyoxyalkylene alkylaryl ether, and a second reagent comprising cholesterol esterase and cholesterol dehydrogenase, said kit further comprising oxidized coenzyme in at least one of the first reagent and the second reagent, and further comprising a polyoxyethylene-polyoxyalkylene alkylaryl ether allowing cholesterol esterase and cholesterol dehydrogenase to act specifically on remnant cholesterol when used in combination with said one kind of surfactant selected from the group consisting of a polyoxyethylene-polyoxybutylene copolymer, a polyoxyethylene styrenated-phenyl ether and a polyoxyalkylene long-chain alkyl ether in at least one of the first reagent and the second reagent.
(19) A kit for the quantitative determination of very low-density lipoprotein remnant cholesterol in a sample which comprises a first reagent comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol dehydrogenase to act specifically on very low-density lipoprotein remnant cholesterol, and a second reagent comprising cholesterol esterase and cholesterol dehydrogenase, said kit further comprising oxidized coenzyme in at least one of the first reagent and the second reagent.
(20) The kit according to any of (16) to (19), wherein the very low-density lipoprotein remnant is intermediate-density lipoprotein.
(21) The kit according to any of (16) to (20), further comprising cyclodextrin or its derivative and/or albumin in at least one of the first reagent and the second reagent.
(22) The kit according to (21), wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.
(23) The kit according to any of (16) to (22), wherein the polyoxyethylene-polyoxyalkylene alkylaryl ether is a polyoxyethylene alkylphenyl ether.

The method of the present invention is described in detail below.

The present invention is characterized in that a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether has an excellent effect as a surfactant for causing enzymes used in the reactions for the determination of cholesterol to act specifically on VLDL remnant-C. That is, these two kinds of surfactants are used in combination to cause cholesterol esterase and cholesterol oxidase or cholesterol dehydrogenase to act specifically on VLDL remnant-C.

The present invention is further characterized in that the above enzymes are inhibited from acting on cholesterol in lipoproteins other than VLDL remnant-C, especially, cholesterol in LDL, by allowing cyclodextrin and its derivative and/or albumin to be present in an aqueous medium in the enzymatic reactions.

According to the present invention, VLDL remnant-C in a sample can be determined by converting, in an aqueous medium, esterified cholesterol in RLP in the sample into free cholesterol by the action of cholesterol esterase in the presence of a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase or cholesterol dehydrogenase to act specifically on VLDL remnant-C in the sample (c) to form hydrogen peroxide from the resulting free cholesterol and free cholesterol in VLDL remnant by the action of cholesterol oxidase or (d) to form reduced coenzyme from the above free cholesterol by the action of cholesterol dehydrogenase in the presence of oxidized coenzyme, and by determining the formed hydrogen peroxide or reduced coenzyme.

In this method, it is preferred to carry out the enzymatic reactions in the presence of cyclodextrin and its derivative and/or albumin because it can prevent the enzymes from acting on cholesterol in lipoproteins other than VLDL remnant.

The method of the present invention can be used to determine VLDL remnant cholesterol in samples such as whole blood, plasma and serum, preferably plasma and serum.

The enzymatic reactions are carried out in an aqueous solution such as a buffer containing a combination of at least two kinds of surfactants of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase or cholesterol dehydrogenase to act specifically on VLDL remnant-C in a sample, cholesterol esterase and cholesterol oxidase or cholesterol dehydrogenase which are necessary for the determination of cholesterol (oxidized coenzyme is necessary when cholesterol dehydrogenase is used) and, if necessary, cyclodextrin and/or albumin. Determination of hydrogen peroxide or reduced coenzyme formed by the reactions is carried out by a method known per se.

The aqueous solution may contain, if necessary, an enzyme activator that is generally used to activate cholesterol esterase and cholesterol oxidase or cholesterol dehydrogenase, a stabilizer, an antiseptic, an interference inhibitor and various kinds of salts for solubilizing proteins such as globulin in a biological sample, as far as they do not affect the specificity of the reaction for determination of VLDL remnant-C.

The reaction for cholesterol ester hydrolysis and the reaction for oxidation or dehydrogenation of free cholesterol may be carried out successively in the presence of the two kinds of surfactants. The reactions may be carried out by contacting a sample with the surfactants in an aqueous solution and then adding the enzymes thereto, but are usually carried out by adding to an aqueous medium all necessary components at a time or after dividing them into two or three groups according to need. More conveniently, a kit for the determination of VLDL remnant-C described below is used.

In the aqueous medium, cholesterol esterase, cholesterol oxidase or cholesterol dehydrogenase is used usually at a concentration of 0.001 to 400 U/ml, preferably 0.01 to 200 U/ml, more preferably 0.05 to 100 U/ml.

In the aqueous medium, the concentration of each of the two kinds of surfactants is preferably 0.001 to 5 (w/v)%, more preferably 0.005 to 2.5 (w/v)%, and particularly preferably 0.05 to 1 (w/v)%.

In the aqueous medium, the concentration of cyclodextrin or its derivative is preferably 0.001 to 5 (w/v)%, more preferably 0.005 to 2.5 (w/v)%, and particularly preferably 0.01 to 1 (w/v)%.

In the aqueous medium, the concentration of albumin is preferably 0.001 to 5 (w/v)%, more preferably 0.005 to 2.5 (w/v)%, and particularly preferably 0.01 to 1 (w/v)%.

The enzymatic reactions are carried out usually at 10 to 50°C, preferably 20 to 40°C, and completed generally in 2 to 30 minutes.

The formed hydrogen peroxide is determined, for example, by subjecting the hydrogen peroxide to reaction with a chromogen which is converted into a dye by oxidation in the presence of peroxidase to form a dye, and measuring the change in the absorbance of the reaction solution at the absorption maximum wavelength of the formed dye. The hydrogen peroxide is also determined by subjecting it to reaction with a chemiluminescent substance such as luminol, isoluminol, lucigenin or acridinium ester to form photon, and determining the formed photon.

The reduced coenzyme can be determined by measuring the absorbance of a solution containing the reduced coenzyme formed by the enzymatic reactions at 300 to 500 nm, preferably 330 to 400 nm, more preferably around 340 nm. The reduced coenzyme can also be determined by subjecting the reduced coenzyme to reaction with a chromogen to form a dye and determining the formed dye. For example, the reduced coenzyme can be determined by subjecting the reduced coenzyme to reaction with diaphorase, an electron carrier (e.g., 1-methoxy-5-methylphenazium methyl sulfate) and a chromogen to form a dye, and measuring the absorbance of the reaction solution at the absorption maximum wavelength of the dye. As the chromogen, a chromogen which is converted into a dye by reduction is used.

The concentration of VLDL remnant-C in a sample can be calculated from a calibration curve showing the relationship between the cholesterol concentration and the amount of hydrogen peroxide or reduced coenzyme previously prepared using samples containing VLDL remnant-C at known concentrations.

As the cholesterol esterase, enzymes that can hydrolyze cholesterol ester, for example, cholesterol esterase and lipoprotein lipase are used. They may be obtained from animals, plants or microorganisms, or produced by genetic engineering techniques, and chemically modified ones can also be used. Examples of the chemically modified enzymes include enzymes that are modified with chemically modifying groups such as a group comprising polyethylene glycol or polypropylene glycol as a main component, a group having a copolymer of polypropylene glycol and polyethylene glycol, a group comprising a water-soluble polysaccharide, a sulfopropyl group, a sulfobutyl group, a polyurethane group and a group having the chelating function. Specifically preferred is an enzyme modified with a group comprising polyethylene glycol as a main component. Examples of the water-soluble polysaccharides include dextran, pullulan and soluble starch.

Examples of reagents for chemical modification (chemical modifiers) include compounds that have both the above chemically modifying group and a functional group or a structure which can react with an amino group, a carboxyl group, a sulfhydryl group or the like of an enzyme. Examples of the functional groups or structures which can react with an amino group of an enzyme include a carboxyl group, an activated ester group (e.g., N-hydroxysuccinimide group), an acid anhydride, an acid chloride, an aldehyde, an epoxide group, 1,3-propanesultone and 1,4-butanesultone. An example of the functional group or structure which can react with a carboxyl group of an enzyme is an amino group. Examples of the groups or structures reactive with a sulfhydryl group of an enzyme include a maleimide group, a disulfide and α-haloester (e.g., α-iodo ester).

Examples of the chemical modifiers are Sunbright VFM-4101, Sunbright MEAC-50HS and Sunbright MEC-50HS which have a group comprising polyethylene glycol as a main component and an N-hydroxysuccinimide group (all produced by NOF Corporation), Sunbright AKM series (e.g., Sunbright AKM-1510), Sunbright ADM series and Sunbright ACM series which have a group comprising polyalkylene glycol as a main component and an acid anhydride structure (all produced by NOF Corporation), EPOX-3400 and M-EPOX-5000 which have a group comprising polyethylene glycol as a main component and an epoxide group (both produced by Sheawater Polymers), diethylenetriamine-N,N,N',N",N"-pentaacetic anhydride which has a group having the chelating function and an acid anhydride structure (DTPA anhydride, Dojindo Laboratories), activated polyurethane P4000 for polyurethane modification (Boehringer Mannheim), dextran T40 for dextran modification and activated TCT (Boehringer Mannheim).

Chemical modification of an enzyme can be carried out, for example, in the following manner.

Cholesterol esterase is dissolved in a buffer of pH 8.0 or higher such as HEPES buffer, and 0.01 to 500-fold molar amount of a chemical modifier is added thereto at 0 to 55°C, followed by stirring for 5 minutes to 5 hours. In the actual enzymatic reaction, this reaction mixture can be used as such, or if necessary, after removal of the unreacted chemical modifier with an ultrafilter, as the chemically modified cholesterol esterase.

The cholesterol oxidase used in the method may be obtained from animals, plants or microorganisms, or produced by genetic engineering techniques, and chemically modified ones can also be used.

The chemically modified enzymes can be prepared by the above method for chemical modification.

As the cholesterol dehydrogenase, any enzymes having the ability to form reduced coenzyme by oxidizing cholesterol in the presence of oxidized coenzyme can be used. They may be obtained from animals, plants or microorganisms, or produced by genetic engineering techniques, and chemically modified ones can also be used. The chemically modified enzymes can be prepared, for example, by the above method for chemical modification using the above chemical modifier.

Examples of the oxidized coenzymes used in the present method for the determination by the use of cholesterol dehydrogenase are NAD, NADP, thio-NAD and thio-NADP.

The polyoxyethylene-polyoxyalkylene alkylaryl ether used in the present invention has the function to cause cholesterol esterase and cholesterol oxidase or cholesterol dehydrogenase to act specifically on VLDL remnant-C when used in combination with one kind of surfactant selected from the group consisting of a polyoxyethylene-polyoxybutylene copolymer, a polyoxyethylene styrenated-phenyl ether and a polyoxyalkylene long-chain alkyl ether used in the present invention. When used alone, it does not necessarily need to have the function to cause cholesterol esterase and cholesterol oxidase or cholesterol dehydrogenase to act specifically on VLDL remnant-C. Hereinafter, the polyoxyethylene-polyoxyalkylene alkylaryl ether used in the present invention is referred to as "surfactant 1".

One kind of surfactant selected from the group consisting of a polyoxyethylene-polyoxybutylene copolymer, a polyoxyethylene styrenated-phenyl ether and a polyoxyalkylene long-chain alkyl ether used in the present invention has the function to cause cholesterol esterase and cholesterol oxidase or cholesterol dehydrogenase to act specifically on VLDL remnant-C when used in combination with the polyoxyethylene-polyoxyalkylene alkylaryl ether used in the present invention. When used alone, it does not necessarily need to have the function to cause cholesterol esterase and cholesterol oxidase or cholesterol dehydrogenase to act specifically on VLDL remnant-C. Hereinafter, one kind of surfactant selected from the group consisting of a polyoxyethylene-polyoxybutylene copolymer, a polyoxyethylene styrenated-phenyl ether and a polyoxyalkylene long-chain alkyl ether used in the present invention is referred to as "surfactant 2".

Examples of the polyoxyethylene-polyoxyalkylene alkylaryl ether include a polyoxyethylene alkylphenyl ether, a polyoxypropylene alkylphenyl ether and a polyoxyethylene-polyoxypropylene alkylphenyl ether. Preferred is a polyoxyethylene-polyoxypropylene alkylphenyl ether.

The alkyl of the polyoxyethylene-polyoxyalkylene alkylaryl ether includes straight-chain or branched alkyl groups having 6 to 15 carbon atoms, such as hexyl, isohexyl, heptyl, isoheptyl, octyl, isooctyl, nonyl, isononyl, decyl, isodecyl, undecyl, isoundecyl, dodecyl, isododecyl, tridecyl, isotridecyl, tetradecyl, isotetradecyl, pentadecyl and isopentadecyl. Specific examples of the polyoxyethylene-polyoxyalkylene alkylaryl ether are Emulgen L-40 (polyoxyethylene-polyoxypropylene alkylphenyl ether), Emulgen 911 (polyoxyethylene alkylphenyl ether) and Emulgen 810 (polyoxyethylene alkylphenyl ether) (all produced by Kao Corporation), and Nonion HS-210 (polyoxyethylene alkylphenyl ether), Nonion HS-215 (polyoxyethylene alkylphenyl ether), Nonion NS-208.5 (polyoxyethylene alkylphenyl ether) and Nonion HS-208 (polyoxyethylene alkylphenyl ether) (all produced by NOF Corporation). Preferred is Emulgen L-40.

The polyoxyethylene-polyoxybutylene copolymer includes random polymers and block polymers of polyoxyethylene and polyoxybutylene, for example, compounds represented by general formula (I):

RO-(C₂H₄O)_{A}-(C₄H₈O)_{B}-(C₂H₄O)_{C}-H (I)

(wherein A, B and C, which may be the same or different, each represent an integer of 1 to 200; and R represents a hydrogen atom or straight-chain or branched alkyl) [hereinafter referred to as Compound (I)]. The alkyl in Compound (I) includes alkyl groups having 1 to 30 carbon atoms, such as methyl, ethyl, propyl, isopropyl, butyl, isobutyl, sec-butyl, tert-butyl, pentyl, isopentyl, neopentyl, hexyl, isohexyl, heptyl, isoheptyl, octyl, isooctyl, nonyl, isononyl, decyl, isodecyl, undecyl, isoundecyl, dodecyl, isododecyl, tridecyl, isotridecyl, tetradecyl, isotetradecyl, pentadecyl, isopentadecyl, hexadecyl, isohexadecyl, heptadecyl, isoheptadecyl, octadecyl, isooctadecyl, nonadecyl, isononadecyl, icosyl, heneicosyl, docosyl (behenyl), tricosyl, tetracosyl, pentacosyl, hexacosyl, heptacosyl, octacosyl, nonacosyl and triacontyl.

Specific examples of Compound (I) include Pronon B-204 and Pronon B-208 (both produced by NOF Corporation). The molecular weight of the polyoxybutylene moiety is preferably 700 or more, more preferably 1,000 or more, and particularly preferably 1,500 or more.

Examples of the polyoxyethylene styrenated-phenyl ether are BLAUNON DSP-9, BLAUNON DSP-12.5, BLAUNON DSP-20, BLAUNON DSP-10, BLAUNON TSP-5, BLAUNON TSP-7.5, BLAUNON TSP-16, BLAUNON TSP-20 and BLAUNON TSP-50, which are commercially available from Aoki Oil Industrial Co., Ltd.

An example of the polyoxyalkylene long-chain branched alkyl ether is UNILUB MT-0620B, which is commercially available from NOF Corporation.

Examples of the cyclodextrin or its derivatives are cyclodextrin, dimethylcyclodextrin, trimethylcyclodextrin, hydroxyethylcyclodextrin, hydroxypropylcyclodextrin, carboxymethylcyclodextrin, glycosylcyclodextrin, maltosylcyclodextrin, cyclodextrin sulfate and cyclodextrin polymer, and preferred are hydroxyethylcyclodextrin and hydroxypropylcyclodextrin.

Examples of the cyclodextrin are α-cyclodextrin, β-cyclodextrin and γ-cyclodextrin.

Examples of the dimethylcyclodextrin are dimethyl-α-cyclodextrin, dimethyl-β-cyclodextrin and dimethyl-γ-cyclodextrin.

Examples of the trimethylcyclodextrin are trimethyl-α-cyclodextrin, trimethyl-β-cyclodextrin and trimethyl-γ-cyclodextrin.

Examples of the hydroxyethylcyclodextrin are hydroxyethyl-α-cyclodextrin, hydroxyethyl-β-cyclodextrin and hydroxyethyl-γ-cyclodextrin.

Examples of the hydroxypropylcyclodextrin are hydroxypropyl-α-cyclodextrin, hydroxypropyl-β-cyclodextrin and hydroxypropyl-γ-cyclodextrin.

Examples of the carboxymethylcyclodextrin are carboxymethyl-α-cyclodextrin, carboxymethyl-β-cyclodextrin and carboxymethyl-γ-cyclodextrin.

Examples of the glycosylcyclodextrin are glycosyl-α-cyclodextrin, glycosyl-β-cyclodextrin and glycosyl-γ-cyclodextrin.

Examples of the maltosylcyclodextrin are maltosyl-α-cyclodextrin, maltosyl-β-cyclodextrin and maltosyl-γ-cyclodextrin.

Examples of the cyclodextrin sulfate are α-cyclodextrin sulfate, β-cyclodextrin sulfate and γ-cyclodextrin sulfate.

An example of the cyclodextrin polymer is a β-cyclodextrin polymer. Two or more kinds of cyclodextrin or derivatives thereof can be used in combination.

Examples of the albumin include albumin obtained from cow, horse, sheep and human, and bovine serum albumin (BSA) is preferred. Albumin produced by genetic engineering techniques can also be used. Two or more kinds of albumin can be used in combination.

Examples of the aqueous media include deionized water, distilled water and a buffer solution, and preferred is a buffer solution.

Examples of the buffers used in the buffer solution include tris(hydroxymethyl)aminomethane buffer, phosphate buffer, borate buffer and Good's buffer. Examples of Good's buffer include 2-morpholinoethanesulfonic acid (MES), bis(2-hydroxyethyl)iminotris(hydroxymethyl)methane (Bis-Tris), N-(2-acetamido)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO) and N-cyclohexyl-3-aminopropanesulfonic acid (CAPS).

The pH of the buffer solution is usually 4 to 10, preferably 5 to 9, and the concentration of the buffer solution is usually 0.001 to 0.5 mol/l, preferably 0.005 to 0.2 mol/l, more preferably 0.01 to 0.1 mol/l.

The chromogens used for the determination of hydrogen peroxide are those which are converted into a dye by oxidation, for example, leuco-type chromogens and oxidative coupling-type chromogens. A leuco-type chromogen is a substance that is converted into a dye by itself in the presence of hydrogen peroxide and a peroxidative substance such as peroxidase. Examples of the leuco-type chromogens are 10-N-carboxymethylcarbamoyl-3,7-bis(dimethylamino)-10H-phenothiazine (CCAP), 10-(N-methylcarbamoyl)-3,7-bis(dimethylamino)-10H-phenothiazine (MCDP), N-(carboxymethylaminocarbonyl)-4,4'-bis(dimethylamino)diphenylamine sodium salt (DA-64), 4,4'-bis(dimethylamino)diphenylamine and bis[3-bis(4-chlorophenyl)methyl-4-dimethylaminophenyl]amine (BCMA).

An oxidative coupling-type chromogen is a chromogen that is converted into a dye by coupling of two compounds by oxidation in the presence of hydrogen peroxide and a peroxidative substance such as peroxidase. Examples of the combinations of the two compounds include combinations of a coupler and aniline and combinations of a coupler and phenol. Examples of the couplers are 4-aminoantipyrine (4-AA) and 3-methyl-2-benzothiazolinone hydrazine. Examples of the anilines include N-(3-sulfopropyl)aniline, N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-(3-sulfopropyl)-3-methylaniline (TOPS), N-(2-hydroxy-3-sulfopropyl)-3,5-dimethoxyaniline (HDAOS), N,N-dimethyl-3-methylaniline, N,N-di(3-sulfopropyl)-3,5-dimethoxyaniline, N-ethyl-N-(3-sulfopropyl)-3-methoxyaniline, N-ethyl-N-(3-sulfopropyl)aniline, N-ethyl-N-(3-sulfopropyl)-3,5-dimethoxyaniline, N-(3-sulfopropyl)-3,5-dimethoxyaniline, N-ethyl-N-(3-sulfopropyl)-3,5-dimethylaniline, N-ethyl-N-(2-hydroxy-3-sulfopropyl)-3-methoxylaniline, N-ethyl-N-(2-hydroxy-3-sulfopropyl) aniline, N-ethyl-N-(3-methylphenyl)-N'-succinylethylenediamine (EMSE), N-ethyl-N-(3-methylphenyl)-N'-acetylethylenediamine and N-ethyl-N-(2-hydroxy-3-sulfopropyl)-4-fluoro-3,5-dimethoxyaniline (F-DAOS). Examples of the phenols include phenol, 4-chlorophenol, 3-methylphenol, 3-hydroxy-2,4,6-triiodobenzoic acid (HTIB).

Peroxidase is used at a concentration of 1 to 100 kU/l. The chromogen is used at a concentration of 0.01 to 10 g/l.

Examples of the chromogens used for the determination of reduced coenzyme include 3-(4,5-dimethyl-2-thiazolyl)-2,5-diphenyl-2H-tetrazolium bromide (MTT), 2-(4-iodophenyl)-3-(4-nitrophenyl)-5-(2,4-disulfophenyl)-2H-tetrazolium monosodium salt (WST-1) and 2-(4-iodophenyl)-3-(2,4-dinitrophenyl)-5-(2,4-disulfophenyl)-2H-tetrazolium monosodium salt (WST-3).

Examples of the enzyme activators include anionic surfactants such as bile acids and alkylsulfonates. Examples of the bile acids are cholic acid, deoxycholic acid, taurocholic acid and chenodeoxycholic acid. Examples of the alkylsulfonates are 1-pentasulfonate, 1-hexasulfonate, 1-heptasulfonate and 1-octasulfonate. Examples of the salts of alkylsulfonate are ammonium salt, lithium salt, sodium salt, potassium salt, magnesium salt and calcium salt.

Examples of the stabilizers are ethylenediaminetetraacetic acid (EDTA), sucrose and calcium chloride.

Examples of the antiseptics include sodium azide and antibiotics.

Examples of the interference inhibitors include ascorbate oxidase to inhibit the effect of ascorbic acid and potassium ferrocyanide to inhibit the effect of bilirubin.

Examples of the salts are lithium chloride, lithium sulfate, sodium chloride, sodium sulfate, potassium chloride, potassium sulfate, magnesium chloride, magnesium sulfate, magnesium acetate, ammonium chloride, magnesium sulfate, magnesium nitrate and calcium nitrate.

Certain embodiments of the method for the determination of VLDL remnant-C of the present invention are illustrated below.

### Method 1

A method which comprises:
(1) carrying out enzymatic reactions in an aqueous medium in the presence of a sample, cholesterol esterase, cholesterol oxidase, surfactant 1 and surfactant 2;
(2) measuring the formed hydrogen peroxide; and
(3) determining the concentration of VLDL remnant-C in the sample from the value measured in (2) and a previously prepared calibration curve.

### Method 2

A method which comprises:
(1) carrying out enzymatic reactions in an aqueous medium in the presence of a sample, cholesterol esterase, oxidized coenzyme, cholesterol dehydrogenase, surfactant 1 and surfactant 2;
(2) measuring the formed reduced coenzyme; and
(3) determining the concentration of VLDL remnant-C in the sample from the value measured in (2) and a previously prepared calibration curve.

### Method 3

A method which comprises:
(1) carrying out enzymatic reactions in an aqueous medium in the presence of a sample, cholesterol esterase, cholesterol oxidase, surfactant 1, surfactant 2, and a cyclodextrin derivative and/or albumin;
(2) measuring the formed hydrogen peroxide; and
(3) determining the concentration of VLDL remnant-C in the sample from the value measured in (2) and a previously prepared calibration curve.

### Method 4

A method which comprises:
(1) carrying out enzymatic reactions in an aqueous medium in the presence of a sample, cholesterol esterase, oxidized coenzyme, cholesterol dehydrogenase, surfactant 1, surfactant 2, and a cyclodextrin derivative and/or albumin;
(2) measuring the formed reduced coenzyme; and
(3) determining the concentration of VLDL remnant-C in the sample from the value measured in (2) and a previously prepared calibration curve.

### Method 5

A method which comprises:
(1) previously mixing a sample with surfactant 2 in an aqueous medium;
(2) carrying out enzymatic reactions in the presence of the mixture of (1), surfactant 1, cholesterol esterase and cholesterol oxidase;
(3) measuring the formed hydrogen peroxide; and
(4) determining the concentration of VLDL remnant-C in the sample from the value measured in (3) and a previously prepared calibration curve.

### Method 6

A method which comprises:
(1) previously mixing a sample with surfactant 2 in an aqueous medium;
(2) carrying out enzymatic reactions in the presence of the mixture of (1), surfactant 1, cholesterol esterase, oxidized coenzyme and cholesterol dehydrogenase;
(3) measuring the formed reduced coenzyme; and
(4) determining the concentration of VLDL remnant-C in the sample from the value measured in (3) and a previously prepared calibration curve.

### Method 7

A method which comprises:
(1) previously mixing a sample with surfactant 2 and a cyclodextrin derivative and/or albumin in an aqueous medium;
(2) carrying out enzymatic reactions in the presence of the mixture of (1), surfactant 1, cholesterol esterase and cholesterol oxidase;
(3) measuring the formed hydrogen peroxide; and
(4) determining the concentration of VLDL remnant-C in the sample from the value measured in (3) and a previously prepared calibration curve.

### Method 8

A method which comprises:
(1) previously mixing a sample with surfactant 2 and a cyclodextrin derivative and/or albumin in an aqueous medium;
(2) carrying out enzymatic reactions in the presence of the mixture of (1), surfactant 1, cholesterol esterase, oxidized coenzyme and cholesterol dehydrogenase;
(3) measuring the formed reduced coenzyme; and
(4) determining the concentration of VLDL remnant-C in the sample from the value measured in (3) and a previously prepared calibration curve.

### Method 9

A method which comprises:
(1) previously mixing a sample with surfactant 1 and surfactant 2 in an aqueous medium;
(2) carrying out enzymatic reactions in the presence of the mixture of (1), cholesterol esterase and cholesterol oxidase;
(3) measuring the formed hydrogen peroxide; and
(4) determining the concentration of VLDL remnant-C in the sample from the value measured in (3) and a previously prepared calibration curve.

### Method 10

A method which comprises:
(1) previously mixing a sample with surfactant 1 and surfactant 2 in an aqueous medium;
(2) carrying out enzymatic reactions in the presence of the mixture of (1), cholesterol esterase, oxidized coenzyme and cholesterol dehydrogenase;
(3) measuring the formed reduced coenzyme; and
(4) determining the concentration of VLDL remnant-C in the sample from the value measured in (3) and a previously prepared calibration curve.

### Method 11

A method which comprises:
(1) previously mixing a sample with surfactant 1, surfactant 2, and a cyclodextrin derivative and/or albumin in an aqueous medium;
(2) carrying out enzymatic reactions in the presence of the mixture of (1), cholesterol esterase and cholesterol oxidase;
(3) measuring the formed hydrogen peroxide; and
(4) determining the concentration of VLDL remnant-C in the sample from the value measured in (3) and a previously prepared calibration curve.

### Method 12

A method which comprises:
(1) previously mixing a sample with surfactant 1, surfactant 2, and a cyclodextrin derivative and/or albumin in an aqueous medium;
(2) carrying out enzymatic reactions in the presence of the mixture of (1), cholesterol esterase, oxidized coenzyme and cholesterol dehydrogenase;
(3) measuring the formed reduced coenzyme; and
(4) determining the concentration of VLDL remnant-C in the sample from the value measured in (3) and a previously prepared calibration curve.

### (Reagent for the Determination of VLDL Remnant-C)

In one embodiment of the invention, the reagent for the determination of VLDL remnant-C comprises cholesterol esterase, cholesterol oxidase, and (a) a polyoxyethylene-polyoxyalkylene alkylaryl ether (surfactant 1) and (b) one kind of surfactant selected from the group consisting of a polyoxyethylene-polyoxybutylene copolymer, a polyoxyethylene styrenated-phenyl ether and a polyoxyalkylene long-chain alkyl ether (surfactant 2) which are capable of causing cholesterol esterase and cholesterol oxidase to act specifically on esterified and free cholesterol in VLDL remnant.

The above reagent may comprise a reagent for the determination of hydrogen peroxide. The reagent for the determination of hydrogen peroxide comprises, for example, peroxidase and a chromogen which is converted into a dye by oxidation.

In another embodiment of the invention, the reagent for the determination of VLDL remnant-C comprises cholesterol esterase, cholesterol dehydrogenase, oxidized coenzyme, and (a) a polyoxyethylene-polyoxyalkylene alkylaryl ether (surfactant 1) and (b) one kind of surfactant selected from the group consisting of a polyoxyethylene-polyoxybutylene copolymer, a polyoxyethylene styrenated-phenyl ether and a polyoxyalkylene long-chain alkyl ether (surfactant 2) which are capable of causing cholesterol esterase and cholesterol dehydrogenase to act specifically on esterified and free cholesterol in VLDL remnant.

This reagent may comprise a reagent for the determination of reduced coenzyme. The reagent for the determination of reduced coenzyme comprises, for example, diaphorase, an electron carrier and a chromogen which is converted into a dye by reduction.

The above reagents may further comprise cyclodextrin or its derivative and/or albumin.

Certain embodiments of the reagent for the determination of VLDL remnant-C of the present invention are illustrated below.

### Reagent 1

A reagent comprising cholesterol esterase, cholesterol oxidase, surfactant 1, surfactant 2 and a reagent for the measurement of hydrogen peroxide

### Reagent 2

A reagent comprising cholesterol esterase, oxidized coenzyme, cholesterol dehydrogenase, surfactant 1 and surfactant 2

### Reagent 3

A reagent comprising cholesterol esterase, oxidized coenzyme, cholesterol dehydrogenase, surfactant 1, surfactant 2 and a reagent for the measurement of reduced coenzyme

### Reagent 4

A reagent comprising cholesterol esterase, cholesterol oxidase, surfactant 1, surfactant 2, a reagent for the measurement of hydrogen peroxide, and a cyclodextrin derivative and/or albumin

### Reagent 5

A reagent comprising cholesterol esterase, oxidized coenzyme, cholesterol dehydrogenase, surfactant 1, surfactant 2, and a cyclodextrin derivative and/or albumin

### Reagent 6

A reagent comprising cholesterol esterase, oxidized coenzyme, cholesterol dehydrogenase, surfactant 1, surfactant 2, a reagent for the measurement of reduced coenzyme, and a cyclodextrin derivative and/or albumin

### (Kit for the Determination of VLDL Remnant-C)

The reagent for the determination of VLDL remnant-C of the present invention can be preserved, distributed and used in the form of a kit. The kit may be composed of two reagents or three reagents, and preferred is a kit composed of two reagents.

In the kit composed of two reagents (a first reagent and a second reagent), cholesterol esterase, and cholesterol oxidase or cholesterol dehydrogenase may be contained in separate reagents, but are preferably contained in the same reagent, specifically preferably in the second reagent. When these enzymes are contained in separate reagents, cholesterol esterase is contained in the first reagent, and cholesterol oxidase or cholesterol dehydrogenase in the second reagent.

One kind of surfactant selected from the group consisting of a polyoxyethylene-polyoxybutylene copolymer, a polyoxyethylene styrenated-phenyl ether and a polyoxyalkylene long-chain alkyl ether may be contained in either the first reagent or the second reagent, but is preferably contained in the first reagent.

A polyoxyethylene-polyoxyalkylene alkylaryl ether is contained in either the first reagent or the second reagent. Cyclodextrin or its derivative and/or albumin is contained in at least one of the first reagent and the second reagent, and is preferably contained in the first reagent. Oxidized coenzyme is contained in at least one of the first reagent and the second reagent. A reagent for the determination of hydrogen peroxide is contained in at least one of the first reagent and the second reagent, but when the reagent comprises an oxidative coupling-type chromogen, the two compounds thereof are preferably contained in separate reagents, respectively. A reagent for the determination of reduced coenzyme is contained in at least one of the first reagent and the second reagent.

Certain embodiments of the kit are illustrated below.

### Kit 1

### First reagent

A reagent comprising surfactant 2 and a reagent for the determination of hydrogen peroxide

### Second reagent

A reagent comprising surfactant 1, cholesterol esterase, cholesterol oxidase and a reagent for the determination of hydrogen peroxide

### Kit 2

### First reagent

A reagent comprising surfactant 2

### Second reagent

A reagent comprising surfactant 1, cholesterol esterase, oxidized coenzyme and cholesterol dehydrogenase

### Kit 3

### First reagent

A reagent comprising surfactant 2

### Second reagent

A reagent comprising surfactant 1, cholesterol esterase, oxidized coenzyme, cholesterol dehydrogenase and a reagent for the determination of reduced coenzyme

### Kit 4

### First reagent

A reagent comprising surfactant 2, a reagent for the determination of hydrogen peroxide, and cyclodextrin or its derivative and/or albumin

### Second reagent

A reagent comprising surfactant 1, cholesterol esterase, cholesterol oxidase and a reagent for the determination of hydrogen peroxide

### Kit 5

### First reagent

A reagent comprising surfactant 2, and cyclodextrin or its derivative and/or albumin

### Second reagent

A reagent comprising surfactant 1, cholesterol esterase, oxidized coenzyme and cholesterol dehydrogenase

### Kit 6

### First reagent

A reagent comprising surfactant 2, and cyclodextrin or its derivative and/or albumin

### Second reagent

A reagent comprising surfactant 1, cholesterol esterase, oxidized coenzyme, cholesterol dehydrogenase and a reagent for the determination of reduced coenzyme

### Kit 7

### First reagent

A reagent comprising surfactant 1, surfactant 2 and a reagent for the determination of hydrogen peroxide

### Second reagent

A reagent comprising surfactant 1, cholesterol esterase, cholesterol oxidase and a reagent for the determination of hydrogen peroxide

### Kit 8

### First reagent

A reagent comprising surfactant 1 and surfactant 2

### Second reagent

A reagent comprising surfactant 1, cholesterol esterase, oxidized coenzyme and cholesterol dehydrogenase

### Kit 9

### First reagent

A reagent comprising surfactant 1 and surfactant 2

### Second reagent

A reagent comprising surfactant 1, cholesterol esterase, oxidized coenzyme, cholesterol dehydrogenase and a reagent for the determination of reduced coenzyme

### Kit 10

### First reagent

A reagent comprising surfactant 1, surfactant 2, a reagent for the determination of hydrogen peroxide, and cyclodextrin or its derivative and/or albumin

### Second reagent

A reagent comprising surfactant 1, cholesterol esterase, cholesterol oxidase and a reagent for the determination of hydrogen peroxide

### Kit 11

### First reagent

A reagent comprising surfactant 1, surfactant 2, and cyclodextrin or its derivative and/or albumin

### Second reagent

A reagent comprising surfactant 1, cholesterol esterase, oxidized coenzyme and cholesterol dehydrogenase

### Kit 12

### First reagent

A reagent comprising surfactant 1, surfactant 2, and cyclodextrin or its derivative and/or albumin

### Second reagent

A reagent comprising surfactant 1, cholesterol esterase, oxidized coenzyme, cholesterol dehydrogenase and a reagent for the determination of reduced coenzyme

### Kit 13

### First reagent

A reagent comprising surfactant 1, surfactant 2 and a reagent for the determination of hydrogen peroxide

### Second reagent

A reagent comprising cholesterol esterase, cholesterol oxidase and a reagent for the determination of hydrogen peroxide

### Kit 14

### First reagent

A reagent comprising surfactant 1 and surfactant 2

### Second reagent

A reagent comprising cholesterol esterase, oxidized coenzyme and cholesterol dehydrogenase

### Kit 15

### First reagent

A reagent comprising surfactant 1 and surfactant 2

### Second reagent

A reagent comprising cholesterol esterase, oxidized coenzyme, cholesterol dehydrogenase and a reagent for the determination of reduced coenzyme

### Kit 16

### First reagent

A reagent comprising surfactant 1, surfactant 2, a reagent for the determination of hydrogen peroxide, and cyclodextrin or its derivative and/or albumin

### Second reagent

A reagent comprising cholesterol esterase, cholesterol oxidase and a reagent for the determination of hydrogen peroxide

### Kit 17

### First reagent

A reagent comprising surfactant 1, surfactant 2, and cyclodextrin or its derivative and/or albumin

### Second reagent

A reagent comprising cholesterol esterase, oxidized coenzyme and cholesterol dehydrogenase

### Kit 18

### First reagent

A reagent comprising surfactant 1, surfactant 2, and cyclodextrin or its derivative and/or albumin

### Second reagent

A reagent comprising cholesterol esterase, oxidized coenzyme, cholesterol dehydrogenase and a reagent for the determination of reduced coenzyme

The reagent and the kit for the determination of VLDL remnant-C of the present invention can be used for the determination of VLDL remnant-C and that of IDL-C.

Cholesterol esterase, cholesterol oxidase, oxidized coenzyme, cholesterol dehydrogenase, cyclodextrin or its derivative, albumin, a polyoxyethylene-polyoxyalkylene alkylaryl ether, one kind of surfactant selected from the group consisting of a polyoxyethylene-polyoxybutylene copolymer, a polyoxyethylene styrenated-phenyl ether and a polyoxyalkylene long-chain alkyl ether, a reagent for the determination of hydrogen peroxide and a reagent for the determination of reduced coenzyme which are described in the above description of the method or reagent for the determination of VLDL remnant-C can be used as the components of the reagent and the kit for the determination of VLDL remnant-C of the present invention.

The reagent and the kit for the determination of VLDL remnant-C of the present invention may comprise, according to need, the above-described aqueous medium, enzyme activator, stabilizer, antiseptic, interference inhibitor and various salts for solubilizing proteins such as globulin in a biological sample.

The reagent and the kit for the determination of VLDL remnant-C of the present invention comprise enzymes, two kinds of surfactants, and cyclodextrin or its derivative and/or albumin as a solution in an aqueous medium at the concentrations described in the above description of the method for the determination of VLDL remnant-C.

Certain embodiments of the present invention are illustrated in the following examples. Reagents and apparatus from the following manufacturers were used in the examples: MOPS (Good's buffer, Dojindo Laboratories), TOOS (Trinder's reagent, Dojindo Laboratories), sodium sulfate (Wako Pure Chemical Industries, Ltd.), Emulgen L-40 (polyoxyethylene-polyoxypropylene alkylphenyl ether, Kao Corporation), Pronon B-208 (polyoxyethylene-polyoxybutylene copolymer, NOF Corporation), peroxidase (POD) (Toyobo Co., Ltd.), ascorbate oxidase (AOD) (Asahi Kasei Corporation), 4-aminoantipyrine (Nakalai Tesque, Inc.), lipoprotein lipase (LPL) (Toyobo Co., Ltd.), cholesterol oxidase (CHOD) (Kyowa Hakko Kogyo Co., Ltd.), BLAUNON TSP-50 (polyoxyethylene styrenated-phenyl ether, Aoki Oil Industrial Co., Ltd.), bovine serum albumin (BSA) (Oriental Yeast Co., Ltd.), UNILUB MT-0620B (polyoxyalkylene long-chain branched alkyl ether, NOF Corporation) and hydroxypropyl-β-cyclodextrin (Nihon Shokuhin Kako Co., Ltd.).

### Brief Description of the Drawings

Fig. 1 is a chromatogram of a fraction having a specific gravity of less than 1.019 prepared by ultracentrifugation as analyzed by gel filtration column chromatography.
Fig. 2 is a graph showing the correlation between the method for the determination of VLDL remnant-C which utilizes a combination of ultracentrifugation and gel filtration (the method of Reference Example 1) and the method of Example 9.
Fig. 3 is a graph showing the correlation between the method for the determination of IDL-C by ultracentrifugation (the method of Reference Example 2) and the method of Example 9.

### Best Modes for Carrying Out the Invention

### Example 1 Kit for the Determination of VLDL Remnant-C

A kit for the determination of VLDL remnant-C comprising the following first reagent and second reagent was prepared.

| First reagent | |
|---|---|
| MOPS (pH 6.5) | 20 mmol/l |
| TOOS | 0.3 g/l |
| Sodium sulfate | 1 g/l |
| Emulgen L-40 | 5 g/l |
| Pronon B-208 | 6 g/l |
| Peroxidase (POD) | 10 U/ml |
| Ascorbate oxidase (AOD) | 2 U/ml |

| Second reagent | |
|---|---|
| MOPS (pH 6.8) | 20 mmol/l |
| 4-Aminoantipyrine | 0.5 g/l |
| Emulgen L-40 | 2 g/l |
| Peroxidase (POD) | 10 U/ml |
| Lipoprotein lipase (LPL) | 2 U/ml |
| Cholesterol oxidase | 2 U/ml |

### Example 2 Kit for the Determination of VLDL Remnant-C

A kit for the determination of VLDL remnant-C comprising the following first reagent and second reagent was prepared.

| First reagent | |
|---|---|
| MOPS (pH 6.5) | 20 mmol/l |
| TOOS | 0.3 g/l |
| Sodium sulfate | 1 g/l |
| BLAUNON TSP-50 | 7 g/l |
| BSA | 1 g/l |
| Peroxidase (POD) | 10 U/ml |
| Ascorbate oxidase (AOD) | 2 U/ml |

| Second reagent | |
|---|---|
| MOPS (pH 6.8) | 20 mmol/l |
| 4-Aminoantipyrine | 0.5 g/l |
| Emulgen L-40 | 2 g/l |
| Peroxidase (POD) | 10 U/ml |
| Lipoprotein lipase (LPL) | 2 U/ml |
| Cholesterol oxidase | 2 U/ml |

### Example 3 Kit for the Determination of VLDL Remnant-C

A kit for the determination of VLDL remnant-C comprising the following first reagent and second reagent was prepared.

| First reagent | |
|---|---|
| MOPS (pH 6.8) | 20 mmol/l |
| TOOS | 0.3 g/l |
| Sodium sulfate | 1 g/l |
| UNILUB MT-0620B | 6 g/l |
| Peroxidase (POD) | 10 U/ml |
| Ascorbate oxidase (AOD) | 2 U/ml |

| Second reagent | |
|---|---|
| MOPS (pH 6.8) | 20 mmol/l |
| 4-Aminoantipyrine | 0.5 g/l |
| Emulgen L-40 | 2 g/l |
| Peroxidase (POD) | 10 U/ml |
| Lipoprotein lipase (LPL) | 2 U/ml |
| Cholesterol oxidase | 2 U/ml |

### Example 4 Kit for the Determination of VLDL Remnant-C

A kit for the determination of VLDL remnant-C comprising the following first reagent and second reagent was prepared.

| First reagent | |
|---|---|
| MOPS (pH 6.5) | 20 mmol/l |
| TOOS | 0.3 g/l |
| Sodium sulfate | 1 g/l |
| Emulgen L-40 | 5 g/l |
| Pronon B-208 | 6 g/l |
| Hydroxypropyl-β-cyclodextrin | 3 g/l |
| Peroxidase (POD) | 10 U/ml |
| Ascorbate oxidase (AOD) | 2 U/ml |

| Second reagent | |
|---|---|
| MOPS (pH 6.8) | 20 mmol/l |
| 4-Aminoantipyrine | 0.5 g/l |
| Emulgen L-40 | 2 g/l |
| Peroxidase (POD) | 10 U/ml |
| Lipoprotein lipase (LPL) | 2 U/ml |
| Cholesterol oxidase | 2 U/ml |

### Example 5 Kit for the Determination of VLDL Remnant-C

A kit for the determination of VLDL remnant-C comprising the following first reagent and second reagent was prepared.

| First reagent | |
|---|---|
| MOPS (pH 6.5) | 20 mmol/l |
| TOOS | 0.3 g/l |
| Sodium sulfate | 1 g/l |
| Emulgen L-40 | 5 g/l |
| Pronon B-208 | 6 g/l |
| BSA | 3 g/l |
| Peroxidase (POD) | 10 U/ml |
| Ascorbate oxidase (AOD) | 2 U/ml |

| Second reagent | |
|---|---|
| MOPS (pH 6.8) | 20 mmol/l |
| 4-Aminoantipyrine | 0.5 g/l |
| Emulgen L-40 | 2 g/l |
| Peroxidase (POD) | 10 U/ml |
| Lipoprotein lipase (LPL) | 2 U/ml |
| Cholesterol oxidase | 2 U/ml |

### Example 6 Determination of VLDL Remnant-C

Determination of VLDL remnant-C in fresh serum (40 samples) was carried out on Hitachi-7170 autoanalyzer using the kit of Example 1 in the following manner.

### (1) Preparation of a calibration curve

As standard solutions, a physiological saline (VLDL remnant-C concentration: 0.0 mg/dl), a serum containing VLDL remnant-C at a concentration of 45 mg/dl {the concentration was determined by the method of Yamamura, et al. ["Arteriosclerosis", Japan Atherosclerosis Society, Vol. 29, p. 235 (title No. 98) (2001)]} and serum dilutions prepared by appropriately diluting the serum were used.

A calibration curve was prepared by determining the concentration of VLDL remnant-C on Hitachi-7170 autoanalyzer using the kit of Example 1 in the following manner.

To a reaction cell were added a standard solution (6 µl) and the first reagent (0.18 ml), and the resulting mixture was incubated at 37°C for 5 minutes. The absorbance of the reaction solution (E1) was measured at a main wavelength of 546 nm and a sub-wavelength of 700 nm. Then, the second reagent (0.05 ml) was added to the reaction solution, followed by further incubation at 37°C for 5 minutes, and the absorbance of the reaction solution (E2) was measured at a main wavelength of 546 nm and a sub-wavelength of 700 nm.

### (2) Determination of VLDL remnant-C contained in fresh human serum (40 samples)

Reaction was carried out in the same manner as in (1) using 40 fresh human serum samples in place of the standard solutions, and VLDL remnant-C contained in each of the 40 samples was determined from the absorbance of the reaction solution after the reaction and the calibration curve prepared in (1).

### Example 7 Determination of VLDL Remnant-C

VLDL remnant-C contained in each of the fresh human serum samples (40 samples) used in the determination of Example 6 was determined on Hitachi-7170 autoanalyzer in the same manner as in Example 6, except that the kit of Example 2 was used in place of the kit of Example 1.

### Example 8 Determination of VLDL Remnant-C

VLDL remnant-C contained in each of the fresh human serum samples (40 samples) used in the determination of Example 6 was determined on Hitachi-7170 autoanalyzer in the same manner as in Example 6, except that the kit of Example 3 was used in place of the kit of Example 1.

### Example 9 Determination of VLDL Remnant-C

VLDL remnant-C contained in each of the fresh human serum samples (40 samples) used in the determination of Example 6 was determined on Hitachi-7170 autoanalyzer in the same manner as in Example 6, except that the kit of Example 4 was used in place of the kit of Example 1.

### Example 10 Determination of VLDL Remnant-C

VLDL remnant-C contained in each of the fresh human serum samples (40 samples) used in the determination of Example 6 was determined on Hitachi-7170 autoanalyzer in the same manner as in Example 6, except that the kit of Example 5 was used in place of the kit of Example 1.

### Reference Example 1 Determination of VLDL Remnant-C by the Method of Yamamura, et al.

VLDL remnant-C in each of the fresh serum samples (40 samples) used in the determination of Example 6 was determined by a combination of ultracentrifugation and gel filtration according to the method of Yamamura, et al. ["Arteriosclerosis", Japan Atherosclerosis Society, Vol. 29, p. 235 (title No. 98) (2001)] in the following manner.

Each sample was subjected to ultracentrifugation to separate a supernatant fraction having a specific gravity of less than 1.019. That is, 2 ml of the sample was added to an ultracentrifuge tube, and 2 ml of a specific gravity-adjusting solution [a physiological saline containing KBr (37.9 g/l)] was added thereto to adjust the specific gravity to 1.019. Then, this tube was placed in a rotor (Hitachi 50.4 TI rotor) and subjected to ultracentrifugation at 35,000 rpm for 20 hours. After the completion of the ultracentrifugation, the separated supernatant was obtained using a tube slicer.

The obtained supernatant contains CM, VLDL and VLDL remnant (the VLDL remnant contains IDL).

The supernatant containing CM, VLDL and VLDL remnant obtained above was separated by gel filtration column chromatography accompanied by coloring reaction system under the following conditions to determine cholesterol. HPLC system : Tosoh Corporation's system
Column: Superose HR6 column (Pharmacia)
Eluent: 0.15 mol/l aqueous solution of sodium chloride containing EDTA (1 mmol/l) (pH 7.4)
Coloring reagent: Determiner L TC (R1 + R2; Kyowa Medex Co., Ltd.)
Flow rate: 0.5 ml/minute (eluent); 0.25 ml/minute (coloring reagent)
Coloring reaction temperature: 37°C
Detection wavelength: 550 nm
Amount of a sample: 3 µl

The concentration of cholesterol in the supernatant was determined using Determiner L TC (a kit for the determination of total cholesterol; Kyowa Medex Co., Ltd.).

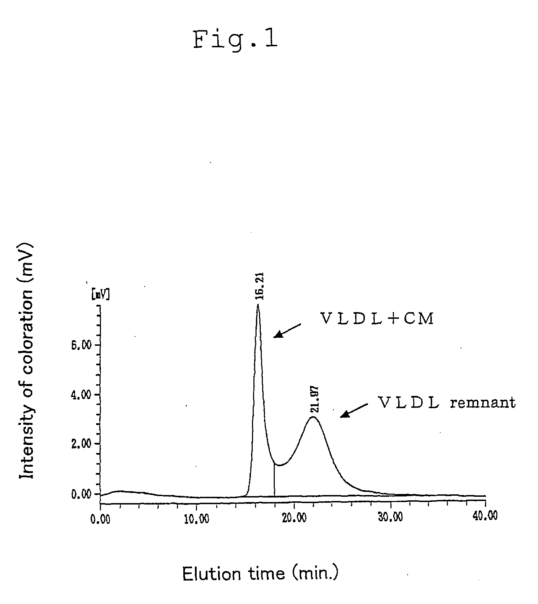

The elution pattern obtained by this gel filtration column chromatography (chromatogram) is shown in Fig. 1. As shown in Fig. 1, VLDL and CM were eluted first and then VLDL remnant.

### Example 11

The amount of VLDL remnant-C in the supernatant prepared by ultracentrifugation was calculated by multiplying the previously determined cholesterol concentration in the supernatant by the volume of the supernatant and the relative area ratio of the VLDL remnant part of the chromatogram in Fig. 1, and the concentration of VLDL remnant-C in the sample was determined.

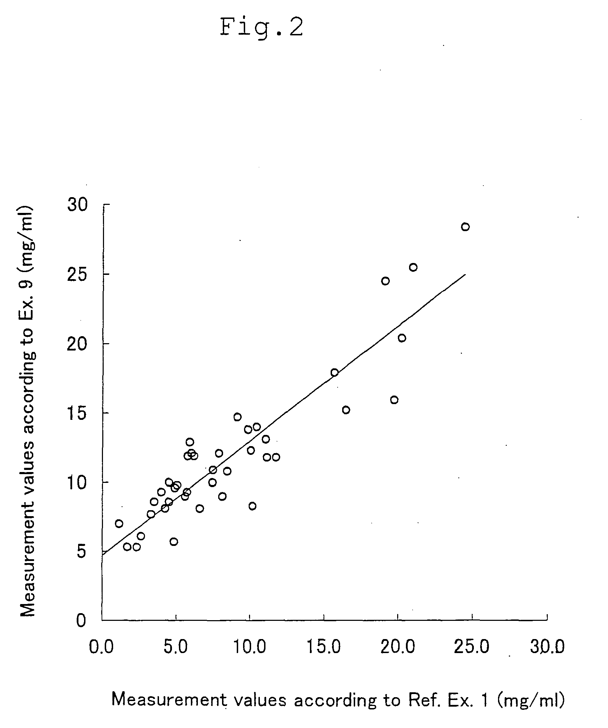

This series of operations from the preparation of a supernatant having a specific gravity of less than 1.019 from a sample by ultracentrifugation through the determination of the concentration of VLDL remnant-C in the sample was carried out on each of the 40 samples. As a result of examining the correlation between the method of Reference Example 1 and the method of Examples 6 to 10, the correlation coefficient between these methods shown in Table 1 was obtained. Fig. 2 shows the correlation between the method of Reference Example 1 and the method of Example 9 (correlation formula: Y=0.83X + 4.7; correlation coefficient: r=0.91). Thus, a good correlation between these methods revealed that VLDL remnant-C in a sample can be determined by the method of the present invention.

### Reference Example 2 Determination of IDL-C by Ultracentrifugation

Each of the fresh serum samples (40 samples) used in the determination of Example 6 was subjected to ultracentrifugation to obtain a fraction having a specific gravity of 1.006 to 1.019 (IDL fraction). The concentration of cholesterol in the obtained IDL fraction was determined using Determiner L TC (a kit for the determination of total cholesterol; Kyowa Medex Co., Ltd.).

### Example 12

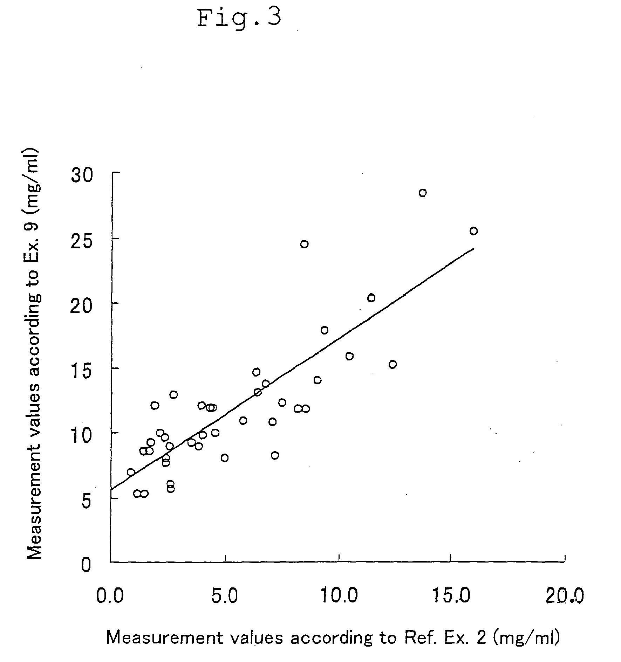

Measurement was carried out using the reagents of Examples 1 to 5 in the same manner as in Example 6, except that a calibration curve was preparead using, as a standard solution, a serum in which the concentration of cholesterol in the fraction having a specific gravity of 1.006 to 1.019 is 23 mg/dl. As a result of examining the correlation between the method of Reference Example 2 and the method of Examples 6 to 10, the correlation coefficient between these methods shown in Table 1 was obtained. Fig. 3 shows the correlation between the method of Reference Example 2 and the method of Example 9 (correlation formula: Y=1.20X + 5.6; correlation coefficient: r=0.83). Thus, a good correlation between these methods revealed that IDL-C in a sample can be determined by the method of the present invention.

**Table 1**

| Kit for measurement (Method for measurement) | Correlation coefficient (Correlation between methods of Exs. and methods of Ref. Exs.) | |
|---|---|---|
| | Ref. Ex. 1 (VLDL remnant-C) | Ref. Ex. 2 (IDL-C) |
| Ex. 1 (Ex. 6) | 0.87 | 0.79 |
| Ex. 2 (Ex. 7) | 0.83 | 0.74 |
| Ex. 3 (Ex. 8) | 0.69 | 0.64 |
| Ex. 4 (Ex. 9) | 0.91 | 0.83 |
| Ex. 5 (Ex. 10) | 0.92 | 0.84 |

As a result of Examples 11 and 12, it was revealed that VLDL remnant-C in a sample and IDL-C in a sample can be determined by the method of the present invention.

### Reference Example 3 Reaction-Inhibiting Effect of Cyclodextrin or Its Derivative and Albumin on LDL

The reactivities of the kits of Examples 1, 4 and 5 on LDL were examined using an LDL fraction prepared by ultracentrifugation. Reaction was carried out in the same manner as in Example 6, except that an LDL fraction was used in place of a standard solution and, as the first reagent and the second reagent, the first reagent and the second reagent in the kits of Examples 1, 4 and 5 were respectively used. The results are shown in Table 2.

**Table 2**

| Kit for measurement | Additives | | LDL Reaction rate (%) | Absorbance (Abs) |
|---|---|---|---|---|
| Example 1 | HP-β-CD | - | 6.3 | 0.355 |
| | BSA | - | | |
| Example 4 | HP-β-CD | + | 2.1 | 0.360 |
| | BSA | - | | |
| Example 5 | HP-β-CD | - | 2.6 | 0.443 |
| | BSA | + | | |

In Table 2, the LDL reaction rate indicates the proportion of the amount of cholesterol in the LDL fraction as determined by the reaction of the LDL fraction with each kit to the amount of cholesterol as determined by the reaction of the LDL fraction with Determiner L TC (a kit for the determination of total cholesterol; Kyowa Medex Co., Ltd.). In Table 2, the absorbance refers to the absorbance of a reaction solution obtained by the reaction of a standard solution having a known concentration of cholesterol with each kit. As can be seen from Table 2, the reaction with cholesterol in LDL was inhibited by the addition of cyclodextrin or its derivative or albumin. Further, the sensitivity was enhanced by the addition of albumin, as revealed by the rise in absorbance.

### Industrial Applicability

The present invention provides a method, a reagent and a kit for the quantitative determination of VLDL remnant-C which are useful for diagnosis of arteriosclerotic diseases.

## Claims

1. A method for quantitatively determining esterified and free cholesterol in very low-density lipoprotein remnant (hereinafter collectively referred to as very low-density lipoprotein remnant cholesterol) in a sample, which comprises: in the presence of a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase or cholesterol dehydrogenase to act specifically on very low-density lipoprotein remnant cholesterol, allowing, in an aqueous medium containing the sample, (c) cholesterol esterase and cholesterol oxidase or (d) in the presence of oxidized coenzyme, cholesterol esterase and cholesterol dehydrogenase to act on cholesterol in very low-density lipoprotein remnant in the sample to form hydrogen peroxide or reduced coenzyme; and determining the formed hydrogen peroxide or reduce coenzyme.

2. The method according to Claim 1, wherein the very low-density lipoprotein remnant is intermediate-density lipoprotein.

3. The method according to Claim 1 or 2, wherein the enzymatic reactions are carried out in the presence of cyclodextrin or its derivative and/or albumin.

4. The method according to Claim 3, wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.

5. The method according to any of Claims 1 to 4, wherein the polyoxyethylene-polyoxyalkylene alkylaryl ether is a polyoxyethylene-polyoxypropylene alkylphenyl ether.

6. The method according to any of Claims 1 to 5, wherein the determination of hydrogen peroxide is carried out by subjecting the formed hydrogen peroxide to reaction with a chromogen in the presence of peroxidase to form a dye and determining the formed dye.

7. The method according to any of Claims 1 to 5, wherein the determination of reduced coenzyme is carried out by measuring the absorbance of the reaction solution.

8. A reagent for the quantitative determination of very low-density lipoprotein remnant cholesterol in a sample comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase to act specifically on very low-density lipoprotein remnant cholesterol, cholesterol esterase and cholesterol oxidase.

9. The reagent according to Claim 8, further comprising a reagent for the determination of hydrogen peroxide.

10. A reagent for the quantitative determination of very low-density lipoprotein remnant cholesterol comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol dehydrogenase to act specifically on very low-density lipoprotein remnant cholesterol, cholesterol esterase, cholesterol dehydrogenase and oxidized coenzyme.

11. The reagent according to Claim 10, further comprising a reagent for the determination of reduced coenzyme.

12. The reagent according to any of Claims 8 to 11, wherein the very low-density lipoprotein remnant cholesterol is intermediate-density lipoprotein.

13. The reagent according to any of Claims 8 to 12, further comprising cyclodextrin or its derivative and/or albumin.

14. The reagent according to Claim 13, wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.

15. The reagent according to any of Claims 8 to 14, wherein the polyoxyethylene-polyoxyalkylene alkylaryl ether is a polyoxyethylene-polyoxypropylene alkylphenyl ether.

16. A kit for the quantitative determination of very low-density lipoprotein remnant cholesterol in a sample which comprises a first reagent comprising one kind of surfactant selected from the group consisting of a polyoxyethylene-polyoxybutylene copolymer, a polyoxyethylene styrenated-phenyl ether and a polyoxyalkylene long-chain alkyl ether which is capable of causing cholesterol esterase and cholesterol oxidase to act specifically on very low-density lipoprotein remnant cholesterol when used in combination with a polyoxyethylene-polyoxyalkylene alkylaryl ether, and a second reagent comprising cholesterol esterase and cholesterol oxidase, said kit further comprising a polyoxyethylene-polyoxyalkylene alkylaryl ether allowing cholesterol esterase and cholesterol oxidase to act specifically on very low-density lipoprotein remnant cholesterol when used in combination with said one kind of surfactant selected from the group consisting of a polyoxyethylene-polyoxybutylene copolymer, a polyoxyethylene styrenated-phenyl ether and a polyoxyalkylene long-chain alkyl ether in at least one of the first reagent and the second reagent, and further comprising a reagent for the determination of hydrogen peroxide in at least one of the first reagent and the second reagent.

17. A kit for the quantitative determination of very low-density lipoprotein remnant cholesterol in a sample which comprises a first reagent comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol oxidase to act specifically on very low-density lipoprotein remnant cholesterol, and a second reagent comprising cholesterol esterase and cholesterol oxidase, said kit further comprising a reagent for the determination of hydrogen peroxide in at least one of the first reagent and the second reagent.

18. A kit for the quantitative determination of very low-density lipoprotein remnant cholesterol in a sample which comprises a first reagent comprising one kind of surfactant selected from the group consisting of a polyoxyethylene-polyoxybutylene copolymer, a polyoxyethylene styrenated-phenyl ether and a polyoxyalkylene long-chain alkyl ether which is capable of causing cholesterol esterase and cholesterol dehydrogenase to act specifically on very low-density lipoprotein remnant cholesterol when used in combination with a polyoxyethylene-polyoxyalkylene alkylaryl ether, and a second reagent comprising cholesterol esterase and cholesterol dehydrogenase, said kit further comprising oxidized coenzyme in at least one of the first reagent and the second reagent, and further comprising a polyoxyethylene-polyoxyalkylene alkylaryl ether allowing cholesterol esterase and cholesterol dehydrogenase to act specifically on remnant cholesterol when used in combination with said one kind of surfactant selected from the group consisting of a polyoxyethylene-polyoxybutylene copolymer, a polyoxyethylene styrenated-phenyl ether and a polyoxyalkylene long-chain alkyl ether in at least one of the first reagent and the second reagent.

19. A kit for the quantitative determination of very low-density lipoprotein remnant cholesterol in a sample which comprises a first reagent comprising a combination of (a) a polyoxyethylene-polyoxyalkylene alkylaryl ether and (b) one kind of surfactant selected from the group consisting of a polyoxyethylene-polyoxybutylene copolymer, a polyoxyethylene styrenated-phenyl ether and a polyoxyalkylene long-chain alkyl ether which combination is capable of causing cholesterol esterase and cholesterol dehydrogenase to act specifically on very low-density lipoprotein remnant cholesterol, and a second reagent comprising cholesterol esterase and cholesterol dehydrogenase, said kit further comprising oxidized coenzyme in at least one of the first reagent and the second reagent.

20. The kit according to any of Claims 16 to 19, wherein the very low-density lipoprotein remnant is intermediate-density lipoprotein.

21. The kit according to any of Claims 16 to 20, further comprising cyclodextrin or its derivative and/or albumin in at least one of the first reagent and the second reagent.

22. The kit according to Claim 21, wherein the cyclodextrin or its derivative is a compound selected from the group consisting of α-cyclodextrin, β-cyclodextrin, γ-cyclodextrin, dimethyl-α-cyclodextrin, dimethyl-β-cyclodextrin, dimethyl-γ-cyclodextrin, trimethyl-α-cyclodextrin, trimethyl-β-cyclodextrin, trimethyl-γ-cyclodextrin, hydroxyethyl-α-cyclodextrin, hydroxyethyl-β-cyclodextrin, hydroxyethyl-γ-cyclodextrin, hydroxypropyl-α-cyclodextrin, hydroxypropyl-β-cyclodextrin, hydroxypropyl-γ-cyclodextrin, carboxymethyl-α-cyclodextrin, carboxymethyl-β-cyclodextrin, carboxymethyl-γ-cyclodextrin, glycosyl-α-cyclodextrin, glycosyl-β-cyclodextrin, glycosyl-γ-cyclodextrin, maltosyl-α-cyclodextrin, maltosyl-β-cyclodextrin, maltosyl-γ-cyclodextrin, α-cyclodextrin sulfate, β-cyclodextrin sulfate, γ-cyclodextrin sulfate and a β-cyclodextrin polymer.

23. The kit according to any of Claims 16 to 22, wherein the polyoxyethylene-polyoxyalkylene alkylaryl ether is a polyoxyethylene alkylphenyl ether.
